# EUROPEAN PATENT APPLICATION

(11) **EP 3 889 255 A1**
(43) Date of publication of application: **06.10.2021**
(21) Application number: 20166793.8
(22) Date of filing: 30.03.2020
(51) Int. Cl.: C12N 9/22, C12N 9/99, C07D 209/00, C12Q 1/44, A61K 31/381

(54) **SMALL MOLECULE INHIBITORS OF RNA GUIDED ENDONUCLEASES**

(71) Applicant: RIJKSUNIVERSITEIT GRONINGEN, 9712 CP Groningen (NL)
(72) Inventor: HAISMA, Hidde Jacob, 9713 AV Groningen (NL); DÖMLING, Alexander Stephan Siegfried, 9713 AV Groningen (NL); LIU, Bin, 9713 AV Groningen (NL)
(74) Representative: Forstmeyer, Dietmar

(57) **Abstract**

The present invention relates to the use of compounds of formula (I) as inhibitors of RNA guided endonucleases, e.g., Cas9 and Cpfl, and methods for the inhibition of the function of such RNA guided endonucleases, including their use in the treatment and prevention of conditions or diseases.

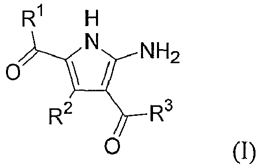

## Description

### Technical Field

The present invention relates to the use of compounds of formula (I) as inhibitors of RNA guided endonucleases, e.g., Cas9 and Cpf1, and methods for the inhibition of the function of such RNA guided endonucleases. Such a regulation of the activity of RNA guided endonucleases is useful in a number of basic research, biomedical or biotechnological applications, including genome editing.

### Background

CRISPR (Clustered Regularly-Interspaced Short Palindromic Repeats) are specialized stretches of DNA which form part of the natural defence mechanisms of bacteria and archaea against invading phages or mobile genetic elements. The most studied CRISPR system employs protein Cas9 (CRISPR-associated protein 9), an RNA-guided endonuclease that acts like a pair of molecular scissors, capable of cleaving double-stranded target DNA in multiple cell types. Cas9 identifies the target sequence by two recognition mechanisms: (i) Watson-Crick base-pairing between the target DNA sequence and guide RNA and (ii) Protospacer Adjacent Motif (PAM) sequence on the target DNA. Upon target recognition as, for example, dictated by binding of a ∼20 nucleotide recombinant "guide RNA" (gRNA) to the target site, Cas9 generates double-stranded breaks (DSB) in the target DNA, e.g. a gene. DSB's produced by Cas9 are most often repaired through the cell's error-prone non-homologous end joining (NHEJ) pathway, which can result in frameshift mutations and gene knockout. Alternatively, homology-directed repair (HDR) at the double-strand break site can allow insertion of a desired sequence.

Known RNA-guided endonuclease variants of CRISPR proteins, include *Streptococcus pyogenes* Cas9 (SpCas9), *Staphylococcus aureus* Cas9 (SaCas9), *Francisella novicida* Cas9 (FnCas9) and Cas12a (FnCas12a; formerly also knows as FnCpf1), *Campylobacter jejuni* Cas9 (CjCas9), Acidaminococcus sp. Cpfl (AsCpfl) and Lachnospiraceae bacterium Cpf1 (LsCpf1). The relative ease of targeting Cas9/Cpf1 to specific genomic loci has enabled the development of revolutionary applications of the CRISPR-Cas9/Cpf1 system in many areas of research and biomedical technologies, including novel therapies to treat genetic disorders.

For example, reports of using the CRISPR-Cas9/Cpf1 system to edit human cells in an experimental setting were published by researchers from the Broad Institute of the Massachusetts Institute of Technology and Harvard, which demonstrated in *in vitro* (laboratory) and animal models of human disease that the technology can be effective in correcting genetic defects. Examples of such diseases include cystic fibrosis, cataracts and Fanconi anemia. The CRISPR-Cas9/Cpfl system has also been applied in the food industry, for example, to engineer probiotic cultures and to vaccinate industrial cultures such as, for example, yogurt, against viruses. In the agricultural industry the CRISPR-Cas9/Cpfl system has also been used, for example, in crops to improve yield, drought tolerance and nutritional properties.

However, the CRISPR-Cas9/Cpfl system is not without its drawbacks. One of the biggest limitations of the CRISPR-Cas9/Cpf1 system is that it is not a hundred percent efficient and produces so-called "off-target effects", where DNA is cut at sites other than the intended target site. These off-target effects are frequently documented and are likely to result from prolonged expression of Cas9/Cpfl in the nuclei of cells, which can then lead to the introduction of unintended mutations, including oncogenic and fatal mutations. An inhibitor of an RNA guided endonuclease (e.g., Cas9 or Cpfl) would therefore provide an effective tool to control the activity of RNA guided endonucleases thereby reducing or prohibiting undesirable off-target effects. Indeed, CRISPR-Cas9/Cpfl-based applications (e.g., genome editing) would benefit from dosable and temporal control (e.g. transcriptional regulation) of the activity of RNA guided endonucleases (e.g., Cas9, Cpfl). Given that the activity of RNA guided endonucleases (e.g., Cas9, Cpfl) is vital to several bacterial processes including immunity, inhibitors of the activity of RNA guided endonucleases (e.g., Cas9, Cpfl) have the potential to afford novel anti-infective agents to counter the ever-growing challenge of antibiotic resistance. An inhibitor of the activity of RNA guided endonucleases (e.g., Cas9, Cpfl) can also have utility as a biodefense agent against CRISPR-based bioterrorism threats.

Hitherto known inhibitors show a number of deficiencies, including low bioavailability/solubility (e.g., lack of cell penetration), lack of selectivity, low potency, limited chemical stability and toxicity (e.g., inhibitors that work at non-physiological concentrations). Reports about potent small molecules that inhibit RNA guided endonucleases (e.g., Cas9, Cpfl) are currently scarce (e.g. Maji et al. A High-Throughput Platform to Identify Small-Molecule Inhibitors of CRISPR-Cas9. 2019, Cell 177, 1067-1079.). In view of the deficits of the prior art, a need exists for small molecule compounds and methods for inhibiting one or more activities of an RNA guided endonuclease (e.g., Cas9, Cpfl). It has therefore been an object to provide compounds compounds, which act as a controllable "off-switch" for the activity of an RNA guided endonuclease (e.g., Cas9, Cpfl) and methods useful for inhibiting the activity of an RNA guided endonuclease (e.g., Cas9, Cpf1).

The present invention provides the use of a compound of formula (I) as inhibitor of the activity of an RNA guided endonuclease (e.g., Cas9, Cpfl) and a method of inhibiting the activity of an RNA guided endonuclease (e.g., Cas9, Cpfl), which include the following advantages: (i) rapid, reversible, dosage, and/or spacio-temporal control of RNA guided endonuclease technologies, e.g. genome editing using the CRISPR-Cas9/Cpfl system; (ii) rapid, tight and reversible binding of the compound to an RNA guided endonuclease (e.g., Cas9, Cpfl); (iii) cell permeability of the compound, including penetration in human cells as well as easy delivery and travel through nuclear membrane; (iv) no cytotoxic effects of the compound at working concentrations; (v) inhibition of activity of naturally occurring RNA guided endonucleases (e.g., SpCas9, SaCas9, FnCas9, FnCas12a, CjCas9, AsCpfl and LsCpfl); and (vi) compatibility with 'split' and drug inducible CRISPR-Cas9/Cpf1 systems.

The present invention relates to the use of a compound of formula (I): 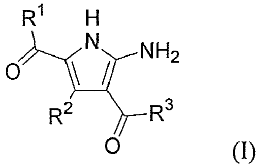 wherein
R¹ is an aryl or heteroaryl group, which groups may optionally be substituted;
R² is an aryl or heteroaryl group, which groups may optionally be substituted; and
R³ is a group of formula 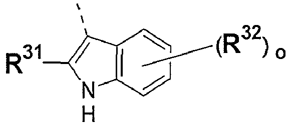
wherein
o is 1 or 2;
R³¹ is a hydrogen atom, deuterium atom, halogen atom, OH, an optionally substituted C₁-C₆alkyl or (C₁-C₆) heteroalkyl;
R³² is independently selected at each occurrence from hydrogen atom, deuterium atom, halogen atom, or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
or a salt thereof, as an inhibitor for the activity of an RNA guided endonuclease in a basic research, biomedical or biotechnological application.

The basic research, biomedical or biotechnological application may be *in vitro* or *in vivo.* In a preferred embodiment, the basic research, biomedical or biotechnological application is *in vitro.* or *in vivo.* In another preferred embodiment the basic research, biomedical or biotechnological application is *in vivo.* These basic research, biomedical or biotechnological application, include gene or genome editing, transcriptional modulation, epigenetic modulation, and genome labelling and/or screening. The compound of formula (I) also has use as a research tool in an *in vitro* or *in vivo* basic research, biomedical or biotechnological application.

Preferably, R¹ may be an optionally substituted aryl group.

Also preferred, R² may be an optionally substituted phenyl group, or an optionally substituted heteroaryl group containing 5, 6, 9 or 10 ring atoms.

Further preferred R¹ may be a group of formula 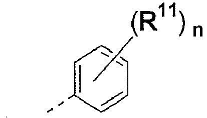 wherein
n is 0, 1, 2, or 3, and
R¹¹ is independently selected at each occurrence from halogen atom, deuterium atom, OH, a C₁₋₆ alkyl group or a C₁₋₆ heteroalkyl group.

R² may preferably be a group of formula 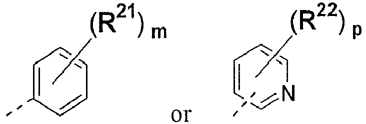 wherein
m is 0, 1, 2, or 3;
p is 0, 1, 2, or 3;
R²¹ is independently selected at each occurrence from halogen atom, deuterium atom, OH, a C₁₋₆ alkyl group or a C₁₋₆ heteroalkyl group; and
R²² is independently selected at each occurrence from halogen atom, deuterium atom, OH, a C₁₋₆ alkyl group or a C₁₋₆ heteroalkyl group.

The basic research, biomedical or biotechnological application may be performed in a cell, the application being performed *in vitro* or *in vivo.*

Preferably, n, m and p each, independently of one another, represents 0, 1 or 2. Moreover preferred, n may be 0 or 1. Also preferred, p may be 0 or 1.

Examples of a preferred embodiment of R¹ include:

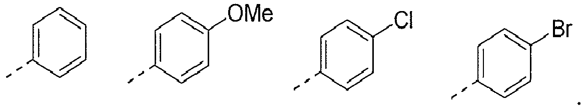

Examples of a preferred embodiment of R² include:

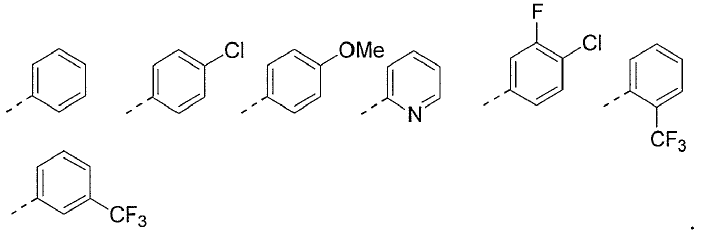

Examples of a preferred embodiment of R³ include:

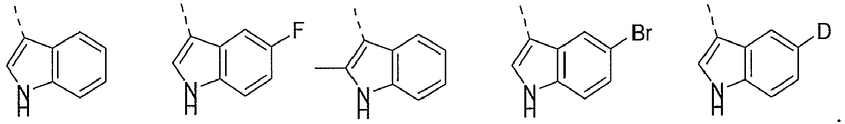

Examples of a preferred compound of formula (I) include compounds D3 and H6 depicted below:

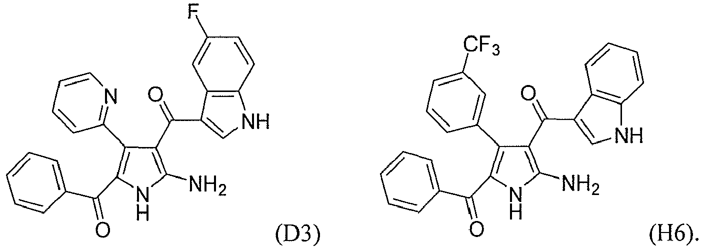

The present invention also relates to a method of inhibiting the activity of an RNA guided endonuclease, the method comprising contacting a compound of formula (I): 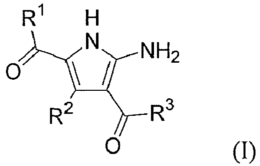 according to any of the embodiments indicated above with an RNA guided endonuclease.

Preferably, the compound of formula (I) and the RNA guided endonuclease are contacted in a cell.

In the present invention, the cell may be a prokaryotic or eukaryotic cell. The prokaryotic cell can be a bacterium cell. The eukaryotic cell can be a human, mammalian, insect, plant, or yeast cell. Preferably, the cell is in an organism.

The RNA guided endonuclease may be a naturally occurring or engineered CRISPR protein, e.g. Cas9 and Cfl1, including SpCas9, SaCas9, FnCas9, FnCas12a, CjCas9, AsCpf1, LsCpf1 and variants thereof. According to a preferred embodiment, the RNA guided endonuclease is a naturally SpCas9, SaCas9, FnCas9, FnCas12a, CjCas9, AsCpfl, or LsCpf1; preferably SpCas9, SaCas9, FnCas9, FnCas12a, or CjCas9; more preferably SpCas9 or SaCas9; and most preferably SpCas9.

The expression alkyl refers to a saturated, straight-chain or branched hydrocarbon group that contains from 1 to 20 carbon atoms, preferably from 1 to 12 carbon atoms, especially from 1 to 6 (e.g. 1, 2, 3 or 4) carbon atoms, for example a methyl, ethyl, propyl, iso-propyl, n-butyl, iso-butyl, sec-butyl, tert-butyl, n-pentyl, iso-pentyl, n-hexyl, 2,2-dimethylbutyl or n-octyl group. Furthermore, the term alkyl refers to groups in which one or more hydrogen atoms have been replaced by a halogen atom (preferably F or Cl) such as, for example, a 2,2,2-trichloroethyl or a trifluoromethyl group.

The expressions alkenyl and alkynyl refer to at least partially unsaturated, straight-chain or branched hydrocarbon groups that contain from 2 to 20 carbon atoms, preferably from 2 to 12 carbon atoms, especially from 2 to 6 (e.g. 2, 3 or 4) carbon atoms, for example an ethenyl (vinyl), propenyl (allyl), iso-propenyl, butenyl, ethinyl, propinyl, butinyl, acetylenyl, propargyl, isoprenyl or hex-2-enyl group. Preferably, alkenyl groups have one or two (especially preferably one) double bond(s), and alkynyl groups have one or two (especially preferably one) triple bond(s). Furthermore, the terms alkenyl and alkynyl refer to groups in which one or more hydrogen atoms have been replaced by a halogen atom (preferably F or Cl).

The expression heteroalkyl refers to an alkyl, alkenyl or alkynyl group in which one or more (preferably 1, 2 or 3) carbon atoms have been replaced by an oxygen, nitrogen, phosphorus, boron, selenium, silicon or sulfur atom (preferably by an oxygen, sulfur or nitrogen atom) or a group of formula SO or SO₂. The expression heteroalkyl furthermore refers to a carboxylic acid or to a group derived from a carboxylic acid, such as, for example, acyl, acylalkyl, alkoxycarbonyl, acyloxy, acyloxyalkyl, carboxyalkylamide or alkoxycarbonyloxy.

Preferably, a heteroalkyl group contains from 1 to 12 carbon atoms and from 1 to 4 hetero atoms selected from oxygen, nitrogen and sulphur (especially oxygen and nitrogen). Especially preferably, a heteroalkyl group contains from 1 to 6 (e.g. 1, 2, 3 or 4) carbon atoms and 1, 2 or 3 (especially 1 or 2) hetero atoms selected from oxygen, nitrogen and sulphur (especially oxygen and nitrogen). The term C₁₋₆ heteroalkyl refers to a heteroalkyl group containing from 1 to 6 carbon atoms and 1, 2 or 3 heteroatoms selected from O, S and/or N (especially O and/or N). The term C₁-₄ heteroalkyl refers to a heteroalkyl group containing from 1 to 4 carbon atoms and 1, 2 or 3 heteroatoms selected from O, S and/or N (especially O and/or N). Furthermore, the term heteroalkyl refers to groups in which one or more hydrogen atoms have been replaced by a halogen atom (preferably F or CI).

Examples of heteroalkyl groups are groups of formulae: R^{a}-O-Y^{a}-, R^{a}-S-Y^{a}-, R^{a}-SO-Y^{a}-, R^{a}-SO₂-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wherein R^{a} being a hydrogen atom, a C₁-C₆ alkyl, a C₂-C₆ alkenyl or a C₂-C₆ alkynyl group; R^{b} being a hydrogen atom, a C₁-C₆ alkyl, a C₂-C₆ alkenyl or a C₂-C₆ alkynyl group; R^{c} being a hydrogen atom, a C₁-C₆ alkyl, a C₂-C₆ alkenyl or a C₂-C₆ alkynyl group; R^{d} being a hydrogen atom, a C₁-C₆ alkyl, a C₂-C₆ alkenyl or a C₂-C₆ alkynyl group and Y^{a} being a direct bond, a C₁-C₆ alkylene, a C₂-C₆ alkenylene or a C₂-C₆ alkynylene group, wherein each heteroalkyl group contains at least one carbon atom and one or more hydrogen atoms may be replaced by fluorine or chlorine atoms.

Specific examples of heteroalkyl groups are methoxy, trifluoromethoxy, ethoxy, n-propyloxy, isopropyloxy, butoxy, *tert*-butyloxy, methoxymethyl, ethoxymethyl, -CH₂CH₂OH, -CH₂OH, methoxyethyl, 1-methoxyethyl, 1-ethoxyethyl, 2-methoxyethyl or 2-ethoxyethyl, methylamino, ethylamino, propylamino, isopropylamino, dimethylamino, diethylamino, isopropylethylamino, methylamino methyl, ethylamino methyl, diisopropylamino ethyl, methylthio, ethylthio, isopropylthio, enol ether, dimethylamino methyl, dimethylamino ethyl, acetyl, propionyl, butyryloxy, acetyloxy, methoxycarbonyl, ethoxycarbonyl, propionyloxy, acetylamino or propionylamino, carboxymethyl, carboxyethyl or carboxypropyl, N-ethyl-N-methylcarbamoyl or N-methylcarbamoyl. Further examples of heteroalkyl groups are nitrile, isonitrile, cyanate, thiocyanate, isocyanate, isothiocyanate and alkylnitrile groups.

The expression cycloalkyl refers to a saturated or partially unsaturated (for example, a cycloalkenyl group) cyclic group that contains one or more rings (preferably 1 or 2), and contains from 3 to 14 ring carbon atoms, preferably from 3 to 10 (especially 3, 4, 5, 6 or 7) ring carbon atoms. The expression cycloalkyl refers furthermore to groups in which one or more hydrogen atoms have been replaced by fluorine, chlorine, bromine or iodine atoms or by OH, =O, SH, =S, NH₂, =NH, N₃ or NO₂ groups, thus, for example, cyclic ketones such as, for example, cyclohexanone, 2-cyclohexenone or cyclopentanone. Further specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, tetraline, cyclopentylcyclohexyl, fluorocyclohexyl or cyclohex-2-enyl group.

The expression heterocycloalkyl refers to a cycloalkyl group as defined above in which one or more (preferably 1, 2, 3 or 4) ring carbon atoms have been replaced by an oxygen, nitrogen, silicon, selenium, phosphorus or sulfur atom (preferably by an oxygen, sulfur or nitrogen atom). A heterocycloalkyl group has preferably 1 or 2 ring(s) containing from 3 to 10 (especially 3, 4, 5, 6 or 7) ring atoms (preferably secected from C, O, N and S). The expression heterocycloalkyl refers furthermore to groups that may be substituted by one or more fluorine, chlorine, bromine or iodine atoms or by one or more OH, =O, SH, =S, NH₂, =NH, N₃ or NO₂ groups. Examples are a piperidyl, prolinyl, imidazolidinyl, piperazinyl, morpholinyl, urotropinyl, pyrrolidinyl, tetrahydrothiophenyl, tetrahydropyranyl, tetrahydrofuryl or 2-pyrazolinyl group and also lactames, lactones, cyclic imides and cyclic anhydrides.

The expression alkylcycloalkyl refers to groups that contain both cycloalkyl and also alkyl, alkenyl or alkynyl groups in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two rings having from 3 to 10 (especially 3, 4, 5, 6 or 7) ring carbon atoms, and one or two alkyl, alkenyl or alkynyl groups (especially alkyl groups) having 1 or 2 to 6 carbon atoms.

The expression heteroalkylcycloalkyl refers to alkylcycloalkyl groups as defined above in which one or more (preferably 1, 2, 3, 4 or 5) carbon atoms have been replaced by an oxygen, nitrogen, silicon, selenium, phosphorus or sulfur atom (preferably by an oxygen, sulfur or nitrogen atom). A heteroalkylcycloalkyl group preferably contains 1 or 2 rings having from 3 to 10 (especially 3, 4, 5, 6 or 7) ring atoms, and one or two alkyl, alkenyl, alkynyl or heteroalkyl groups (especially alkyl or heteroalkyl groups) having from 1 or 2 to 6 carbon atoms. Examples of such groups are alkylheterocycloalkyl, alkylheterocycloalkenyl, alkenylheterocycloalkyl, alkynylheterocycloalkyl, heteroalkylcycloalkyl, heteroalkylheterocycloalkyl and hetero-alkylheterocycloalkenyl, the cyclic groups being saturated or mono-, di- or tri-unsaturated.

The expression aryl refers to an aromatic group that contains one or more rings containing from 5 or 6 to 14 ring carbon atoms, preferably from 5 or 6 to 10 (especially 6) ring carbon atoms. The expression aryl refers furthermore to groups in which one or more hydrogen atoms have been replaced by fluorine, chlorine, bromine or iodine atoms or by OH, SH, NH₂, N₃ or NO₂ groups. Examples are the phenyl, naphthyl, biphenyl, 2-fluorophenyl, anilinyl, 3-nitrophenyl or 4-hydroxyphenyl group.

The expression heteroaryl refers to an aromatic group that contains one or more rings containing from 5 to 14 ring atoms, preferably from 5 to 10 (especially 5 or 6 or 9 or 10) ring atoms, and contains one or more (preferably 1, 2, 3, 4 or 5) oxygen, nitrogen, phosphorus or sulfur ring atoms (preferably O, S or N). The expression heteroaryl refers furthermore to groups in which one or more hydrogen atoms have been replaced by fluorine, chlorine, bromine or iodine atoms or by OH, SH, N₃, NH₂ or NO₂ groups. Examples are pyridyl (e.g. 4-pyridyl), imidazolyl (e.g. 2-imidazolyl), phenylpyrrolyl (e.g. 3-phenylpyrrolyl), thiazolyl, isothiazolyl, 1,2,3-triazolyl, 1,2,4-triazolyl, oxadiazolyl,thiadiazolyl, indolyl, indazolyl, tetrazolyl, pyrazinyl, pyrimidinyl, pyridazinyl, oxazolyl, isoxazolyl, triazolyl, tetrazolyl, isoxazolyl, indazolyl, indolyl, benzimidazolyl, benzoxazolyl, benzisoxazolyl, benzthiazolyl, pyridazinyl, quinolinyl, isoquinolinyl, pyrrolyl, purinyl, carbazolyl, acridinyl, pyrimidyl, 2,3'-bifuryl, pyrazolyl (e.g. 3-pyrazolyl) and isoquinolinyl groups.

The expression aralkyl refers to groups containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are toluene, xylene, mesitylene, styrene, benzyl chloride, o-fluorotoluene, 1H-indene, tetraline, dihydronaphthalene, indanone, phenylcyclopentyl, cumene, cyclohexylphenyl, fluorene and indane. An aralkyl group preferably contains one or two aromatic ring systems (1 or 2 rings) containing from 6 to 10 carbon atoms and one or two alkyl, alkenyl and/or alkynyl groups containing from 1 or 2 to 6 carbon atoms and/or one or two cycloalkyl groups containing 5 or 6 ring carbon atoms.

The expression heteroaralkyl refers to an aralkyl group as defined above in which one or more (preferably 1, 2, 3 or 4) carbon atoms have been replaced by an oxygen, nitrogen, silicon, selenium, phosphorus, boron or sulfur atom (preferably oxygen, sulfur or nitrogen), that is to say to groups containing both aryl or heteroaryl, respectively, and also alkyl, alkenyl, alkynyl and/or heteroalkyl and/or cycloalkyl and/or heterocycloalkyl groups in accordance with the above definitions. A heteroaralkyl group preferably contains one or two aromatic ring systems (1 or 2 rings) containing from 5 or 6 to 10 ring carbon atoms and one or two alkyl, alkenyl and/or alkynyl groups containing 1 or 2 to 6 carbon atoms and/or one or two cycloalkyl groups containing 5 or 6 ring carbon atoms, wherein 1, 2, 3, 4, 5 or 6 of these carbon atoms have been replaced by oxygen, sulfur or nitrogen atoms.

Examples are arylheteroalkyl, arylheterocycloalkyl, arylheterocycloalkenyl, arylalkyl-heterocycloalkyl, arylalkenylheterocycloalkyl, arylalkynylheterocycloalkyl, arylalkylhetero-cycloalkenyl, heteroarylalkyl, heteroarylalkenyl, heteroarylalkynyl, heteroarylheteroalkyl, heteroarylcycloalkyl, heteroarylcycloalkenyl, heteroarylheterocycloalkyl, hetero-arylheterocycloalkenyl, heteroarylalkylcycloalkyl, heteroarylalkylheterocycloalkenyl, hetero-arylheteroalkylcycloalkyl, heteroarylheteroalkylcycloalkenyl and heteroarylheteroalkylhetero-cycloalkyl groups, the cyclic groups being saturated or mono-, di- or tri-unsaturated. Specific examples are a tetrahydroisoquinolinyl, benzoyl, 2- or 3-ethylindolyl, 4-methylpyridino, 2-, 3- or 4-methoxyphenyl, 4-ethoxyphenyl, 2-, 3- or 4-carboxyphenylalkyl group.

The term halogen or halogen atom refers to F, Cl, Br or I.

The expression "optionally substituted" preferably refers to groups in which one, two, three or more hydrogen atoms may have been replaced by fluorine, chlorine, bromine or iodine atoms or by OH, =O, SH, =S, NH₂, =NH, N₃ or NO₂ groups. This expression refers furthermore to groups that may be substituted by one, two, three or more preferably unsubstituted C₁-C₁₀ alkyl, C₂-C₁₀ alkenyl, C₂-C₁₀ alkynyl, C₁-C₁₀ heteroalkyl, C₃-C₁₈ cycloalkyl, C₂-C₁₇ heterocycloalkyl, C₄-C₂₀ alkylcycloalkyl, C₂-C₁₉ heteroalkylcycloalkyl, C₆-C₁₈ aryl, C₁-C₁₇ heteroaryl, C₇-C₂₀ aralkyl or C₂-C₁₉ heteroaralkyl groups. This expression refers furthermore especially to groups that may be substituted by one, two, three or more preferably unsubstituted C₁-C₆ alkyl, C₂-C₆ alkenyl, C₂-C₆ alkynyl, C₁-C₆ heteroalkyl, C₃-C₁₀ cycloalkyl, C₂-C₉ heterocycloalkyl, C₇-C₁₂ alkylcycloalkyl, C₂-C₁₁ heteroalkylcycloalkyl, C₆-C₁₀ aryl, C₁-C₉ heteroaryl, C₇-C₁₂ aralkyl or C₂-C₁₁ heteroaralkyl groups.

If a substituent contains a ring, this ring may be bonded to the respective substituted group via a single or double bond (especially a single bond) or, if the substituted group also contains a ring, the ring of the substituent may also be annulated to the ring of the substituted group.

Preferred substituents are F, Cl, Br, I, OH, =O, NH₂, NO₂, C₁₋₄ alkyl (e.g. -CH3, CF₃), C₁₋₄ heteroalkyl (e.g. -CN, -OMe), cyclopropyl and SO₂NH₂.

Especially preferred substituents are F, Cl, Br, OH, =O, NH₂, CH₂NH₂, NO₂, Me, Ethyl, NMe₂, CONH₂, OMe, CN and CF₃. The most preferred substituents are F and Cl.

According to a preferred embodiment, all alkyl, alkenyl, alkynyl, heteroalkyl, aryl, heteroaryl, cycloalkyl, heterocycloalkyl, alkylcycloalkyl, heteroalkylcycloalkyl, aralkyl and heteroaralkyl groups described herein may optionally be substituted.

When an aryl, heteroaryl, cycloalkyl, alkylcycloalkyl, heteroalkylcycloalkyl, heterocycloalkyl, aralkyl or heteroaralkyl group contains more than one ring, these rings may be bonded to each other via a single or double bond or these rings may be annulated.

A compound provided herein can be synthesized using a variety of methods known in the art, for example as disclosed in Guo et al., Eur. J. Med. Chem. 139 (2017), 633-643 and Frolova et al., Org. Lett. 13 (2011), 1118-1121. The scheme and description below depicts a general route for the preparation of compounds of formula (1).

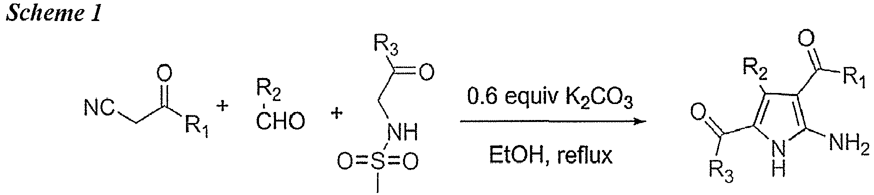

### General synthetic route to compounds of formula (1). Reagents and conditions: aldehydes, cyanoacetic acid derivatives, methylsulfonamidoacetophenone, K₂CO₃, EtOH, reflux, 12 h.

The present invention further provides a pharmaceutical composition comprising one or more compounds of formula (I) as defined herein or a pharmaceutically acceptable ester, prodrug, hydrate, solvate or salt thereof, optionally in combination with a pharmaceutically acceptable carrier.

Small molecule inhibitors of RNA guided endonucleases (e.g., Cas9, Cpfl) of formula (I) as defined herein have potential therapeutic uses for regulating genome editing technologies involving RNA guided endonucleases. Dosable control of the therapeutic activity of RNA guided endonucleases introduced into a subject or cell of a subject is important for effective genome editing therapeutic strategies. Small molecule inhibitors of RNA guided endonucleases can be administered to a subject undergoing RNA guided endonuclease-based gene therapy or any other RNA guided endonuclease-based therapy. In certain embodiments, the subject is a human or mammal. Small molecule inhibitors of RNA guided endonucleases eliminate or reduce undesirable off-target editing and chromosomal translocations when present at high concentrations Furthermore, small molecule inhibitors of RNA guided endonucleases can be used to rapidly terminate constitutively active CRISPR protein, following on-target gene-editing.

Small molecule inhibitors of RNA guided endonucleases (e.g., Cas9, Cpfl) of formula (I) as defined herein can also be used to regulate genome editing technologies in other organisms, including invertebrates, plants, and unicellular organisms (e.g., bacteria). Potential uses include regulating gene drives for entomological and agricultural uses. In addition, it is anticipated that CRISPR protein inhibitors will be valuable tools for antibiotic therapies as they will disrupt bacterial immunity against bacteriophages (or toxic DNA) by interfering with the CRISPR-Cas-based immune surveillance system in bacteria or inhibit bacterial growth.

It is a further object of the present invention to provide a compound of formula (I) as defined herein, a pharmaceutically acceptable ester, prodrug, hydrate, solvate or salt thereof, or a pharmaceutical composition comprising one or more compounds of formula (I) as defined herein or a pharmaceutically acceptable ester, prodrug, hydrate, solvate or salt thereof, optionally in combination with a pharmaceutically acceptable carrier, for the preparation of a medicament for inhibiting RNA guided endonucleases (e.g., Cas9, Cpfl) in the treatment and/or prevention of a bacterial infection; or a genetic disorder, including hereditary diseases such as, for example, sickle cell anaemia, cystic fibrosis, and haemophilia; asthma (e.g. acute and chronic asthma), COPD, allergic rhinitis, erectile dysfunction, pulmonary hypertension, hypertension, T cell dysfunction, atherosclerosis, renal disease, ischemia reperfusion injury, neurodegenerative diseases, wound healing, inflammatory diseases, fibrotic diseases and cancer (e.g., by gene therapy).

A therapeutically effective amount of a compound in accordance with this invention means an amount of compound that is effective to prevent, alleviate or ameliorate symptoms of disease or prolong the survival of the subject being treated. Determination of a therapeutically effective amount is within the skill in the art.

The therapeutically effective amount or dosage of a compound according to this invention can vary within wide limits and may be determined in a manner known in the art. Such dosage may be adjusted to the individual requirements in each particular case including the specific compound being administered, the route of administration, the condition being treated, as well as the patient being treated.

Examples of pharmacologically acceptable salts of sufficiently basic compounds of formula (I) are salts of physiologically acceptable mineral acids like hydrochloric, hydrobromic, sulfuric and phosphoric acid; or salts of organic acids like methane sulfonic, p-toluene sulfonic, lactic, acetic, trifluoroacetic, citric, succinic, fumaric, maleic and salicylic acid. Further, a sufficiently acidic compound of formula (I) may form alkali or earth alkali metal salts, for example sodium, potassium, lithium, calcium or magnesium salts; ammonium salts; or organic base salts, for example methylamine, dimethylamine, trimethylamine, triethylamine, ethylenediamine, ethanolamine, choline hydroxide, meglumine, piperidine, morpholine, tris-(2-hydroxyethyl)amine, lysine or arginine salts; all of which are also further examples of salts of formula (1). Compounds of formula (I) may be solvated, especially hydrated. The hydratization/hydration may occur during the process of production or as a consequence of the hygroscopic nature of the initially water free compounds of formula (1). The solvates and/or hydrates may e.g. be present in solid or liquid form.

It should be appreciated that certain compounds of formula (I)may have tautomeric forms from which only one might be specifically mentioned or depicted in the following description, different geometrical isomers (which are usually denoted as cis/trans isomers or more generally as (E) and (Z) isomers) or different optical isomers as a result of one or more chiral carbon atoms (which are usually nomenclatured under the Cahn-Ingold-Prelog or R/S system). All these tautomeric forms, geometrical or optical isomers (as well as racemates and diastereomers) and polymorphous forms are included in the invention. Since the compounds of formula (I)may contain asymmetric C-atoms, they may be present either as achiral compounds, mixtures of diastereomers, mixtures of enantiomers or as optically pure compounds. The present invention comprises both all pure enantiomers and all pure diastereomers, and also the mixtures thereof in any mixing ratio.

The therapeutic use of compounds according to formula (1), their pharmacologically acceptable salts, solvates and hydrates, respectively, as well as formulations and pharmaceutical compositions also lie within the scope of the present invention. Such formulations and pharmaceutical compositions may also contain a pro-drug, which is composed of a compound of formula (I)and at least one pharmacologically acceptable protective group which will be cleaved off under physiological conditions, such as an alkoxy-, arylalkyloxy-, acyl-, acyloxymethyl group (e.g. pivaloyloxymethyl), an 2-alkyl-, 2-aryl- or 2-arylalkyl-oxycarbonyl-2-alkylidene ethyl group or an acyloxy group as defined herein, e.g. ethoxy, benzyloxy, acetyl or acetyloxy or, especially for a compound of formula (I)carrying a hydroxy group (-OH): a sulfate, a phosphate (-OPO₃ or -OCH₂OPO₃) or an ester of an amino acid.

As used herein, the term pharmaceutically acceptable ester especially refers to esters which hydrolyse in vivo and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters include, but are not limited to, formates, acetates, propionates, butyrates, acrylates and ethylsuccinates.

As mentioned above, therapeutically useful agents that contain one or more compounds of formula (1), solvates, salts or formulations thereof, are also comprised in the scope of the present invention. In general, a compound of formula (I) can be administered by using the known and acceptable modes known in the art, either alone or in combination with any other therapeutic agent.

For oral administration such therapeutically useful agents can be administered by one of the following routes: oral, e.g. as tablets, dragees, coated tablets, pills, semisolids, soft or hard capsules, for example soft and hard gelatine capsules, aqueous or oily solutions, emulsions, suspensions or syrups, parenteral including intravenous, intramuscular and subcutaneous injection, e.g. as an injectable solution or suspension, rectal as suppositories, by inhalation or insufflation, e.g. as a powder formulation, as microcrystals or as a spray (e.g. liquid aerosol), transdermal, for example via an transdermal delivery system (TDS) such as a plaster containing the active ingredient or intranasal. For the production of such tablets, pills, semisolids, coated tablets, dragees and hard, e.g. gelatine, capsules the therapeutically useful product may be mixed with pharmaceutically inert, inorganic or organic excipients as are e.g. lactose, sucrose, glucose, gelatine, malt, silica gel, starch or derivatives thereof, talc, stearinic acid or their salts, dried skim milk, and the like. For the production of soft capsules one may use excipients as are e.g. vegetable, petroleum, animal or synthetic oils, wax, fat, polyols. For the production of liquid solutions, emulsions or suspensions or syrups one may use as excipients e.g. water, alcohols, aqueous saline, aqueous dextrose, polyols, glycerin, lipids, phospholipids, cyclodextrins, vegetable, petroleum, animal or synthetic oils. Especially preferred are lipids and more preferred are phospholipids (preferred of natural origin; especially preferred with a particle size between 300 to 350 nm) preferred in phosphate buffered saline (pH = 7 to 8, preferred 7.4). For suppositories one may use excipients as are e.g. vegetable, petroleum, animal or synthetic oils, wax, fat and polyols. For aerosol formulations one may use compressed gases suitable for this purpose, as are e.g. oxygen, nitrogen and carbon dioxide. The pharmaceutically useful agents may also contain additives for conservation, stabilization, e.g. UV stabilizers, emulsifiers, sweetener, aromatizers, salts to change the osmotic pressure, buffers, coating additives and antioxidants.

In general, in the case of oral or parenteral administration to adult humans weighing approximately 80 kg, a daily dosage of about 10 mg to about 10,000 mg, preferably from about 20 mg to about 1,000 mg, should be appropriate, although the upper limit may be exceeded when indicated. The daily dosage can be administered as a single dose or in divided doses, or for parenteral administration, it may be given as continuous infusion or subcutaneous injection.

As used herein, "comprising", "including", "containing", "characterized by", and grammatical equivalents thereof are inclusive or open-ended terms that do not exclude additional, unrecited elements or method steps. Yet, "Comprising", etc. is also to be interpreted as including the more restrictive terms "consisting essentially of" and "consisting of', respectively.

The compound of formula (I)is described herein using a general formula and it should be understood that combinations of substituents that do not result in stable compounds, i.e., compounds that can be isolated, characterized and tested for biological activity, are excluded. Further, the general formula (I) of the compound described herein includes variables such as, *e.g.* R¹-R³. Unless otherwise specified, each variable within such a formula is defined independently of any other variable, and any variable that occurs more than one time in a formula is defined independently at each occurrence. Thus, for example, if a group is shown to be substituted with 0-2 R^{*}, the group may be unsubstituted, or substituted with 1 or 2 group(s) R^{*}, wherein R^{*} at each occurrence is selected independently from the corresponding definition of R^{*}. Also, combinations of substituents and/or variables are permissible only if such combinations result in stable compounds, *i.e.,* compounds that can be isolated, characterized and tested for biological activity.

As used herein, "consisting of" excludes any element, step, or ingredient not specified in the claim.

When trade names are used herein, it is intended to independently include the trade name product formulation, the generic drug, and the active pharmaceutical ingredient(s) of the trade name product.

In general, unless defined otherwise, technical and scientific terms used herein have the same meaning as commonly understood by one of ordinary skill in the art to which this invention belongs, and are consistent with general textbooks and dictionaries.

### Brief Description of the Figures

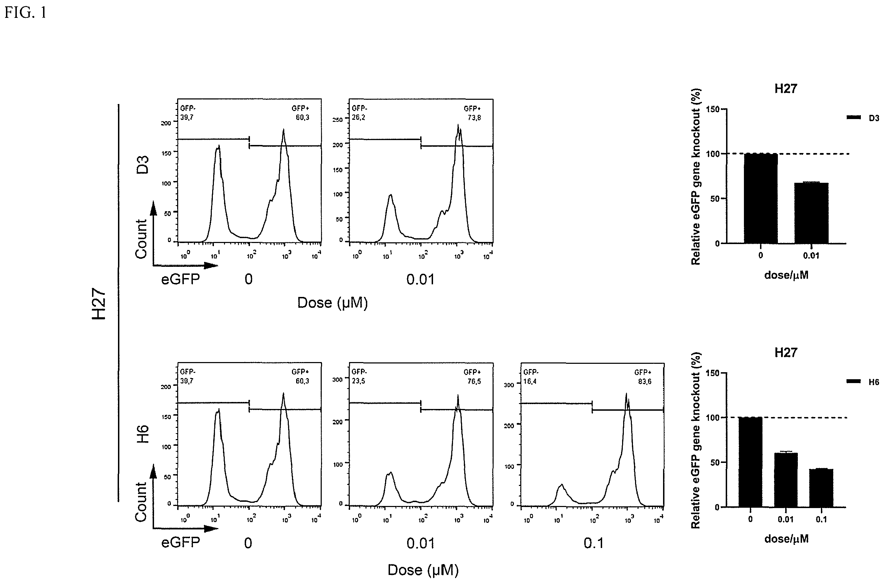

**Figure 1****.** Dose dependent inhibition of genome editing in H27 cells by compounds according to general formula (I).

The present invention is now further illustrated by the following examples from which features, embodiments and advantages of the present invention may be taken.

### EXAMPLES

### A. Materials and Methods

### 1. Assays for high throughput screening

### (a) Cell viability assay

The effects of compounds on cell variability were detected using an MTS assay. Briefly, 3x10³ cells /well were seeded in 96-well plates and cultured overnight, following by incubation of the corresponding compounds for 48 hr. The next day, cells were incubated with MTS for 90 min at 37°C according to the instruction of CellTiter 96 AQueous One Solution Reagent (Promega, Madison, USA). The absorbance values were measured using a Synergy H1 plate reader (BioTek, Winooski, USA) at the wavelength of 490 nm by. Three independent experiments were performed in triplicate.

### (b) eGFP expression detection assay

AdV-Cas9 and AdV-gRNA were used to infect H27 cells to screen potential compounds that affect the EGFP gene knockout efficiency using CRISPR/Cas9. 1000 H27 cells per well were seeded in a black Advanced TC™ 96-wells plate (Greiner Bio-One) to avoid the interference of light. After 24 hr incubation, the medium was changed by medium containing compounds with final concentration of 10 µM. The AdV-Cas9 and AdV-gRNA were mixed in a ratio of 1:1 were used to infect the H27 cells in a quantity of 30 TU/well. Cells were sub-cultured for 12 days for EGFP protein degradation. Subsequently, cells were washed and fluorescence was determined by a Synergy H1 plate reader (BioTek, Winooski, USA) using the excitation wavelength of 485nm and emission wavelength of 525nm.

Additionally, mock transduced H27 cells acted as a control to detect the influence of the different compounds on the expression of endogenous EGFP. Similar with knock-out experiments, H27 cells were seeded in a density of 1000 cells/well in a black 96-well plate. Following by the treatment of compounds with final concentration of 10 µM. After three days, a Synergy H1 plate reader was performed to determine the fluorescent values of EGFP using the same excitation and emission wavelength as knock-out experiments.

### Example 1 - Investigation of cell viability

A cell viability assay was performed with compounds of formula (1) in H27 cells using the above described MTS assay. All compounds were diluted to a final concentration of 10 µM in DMSO from a 10 mM stock solution. The observed cell viability of cells treated with compounds of formula (1) was generally found to be good, i.e. >90% as compared to the non-treatment group.

### Example 2 - Investigation of eGFP expression

Since the compounds may influence endogenous eGFP expression, the interference of the compounds on endogenous eGFP expression was determined with the above described eGFP expression detection assay. It was found that most of the investigated compounds did not significantly interfere with endogenous eGFP expression.

In the same way, gene knockout efficiency of the compounds was measured. It was found that the CRISPR/Cas9 gene editing efficiency was altered in cells treated with compounds.

### Example 3 - Evaluation of example compounds

The following two example compounds (D3 and H6) of a compound according to general formula(I): 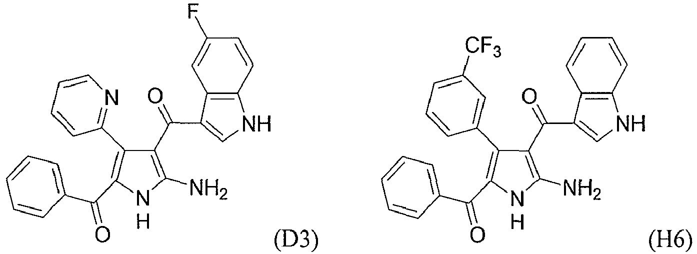 which showed good cell viability (cf. Example 1), assay, no significant interference with endogenous eGFP expression (cf. Example 2), and promising gene knockout efficiency (cf. Example 2), were selected for further testing in methods according to the present invention for their utility as inhibitors of RNA guided endonucleases using the currently most widely used SpCas9.

### 3.1 Inhibition of genome editing by D3 and H6 with different doses

The gene knockout efficiency inhibited by D3 and H6 was tested with different doses using flow cytometric analysis. For EGFP knock-out assays, cells were collected using trypsinization and washed twice with FACS buffer (PBS plus 1% FBS). The population of EGFP⁺ cells was quantified using FACS Calibur flow cytometer (BD, Franklin Lakes, USA). All the experiments were performed at the Central Flow cytometry Unit (University Medical Center Groningen). Data were analysed by FlowJo 7.2.2 software (Tree Star). It was found that D3 and H6 inhibited genome editing in a dose dependent manner. The results are shown in FIG. 1.

### 3.2 Effects of D3 and H6 on cell viability and endogenous eGFP expression with different doses

The effects of different concentrations of D3 and H6 on cell viability and endogenous eGFP expression with different doses were determined by MTS and flow cytometric analysis in accordance with the methods described above. The experiments were performed in H27 and HEK.EGFP^{TetO.KRAB} (see, References 1, 2). While a good cell viability of both H27 and HEK.EGFP^{TetO.KRAB} was observed for D3 in the concentration range of 0.01 to 100 µM, cell viability was reduced for H6 in the concentration range of 50 to 100 µM. No significant interference with endogenous eGFP expression in H27 and HEK.EGFP^{TetO.KRAB} was observed for D3 and H6 at concentrations of 0.01 µM and 0.1 µM, respectively.

### 3.3 Effects of D3 and H6 on gRNA expression level

The effects of D3 and H6 on guide RNA expression level in H27 and HEK.EGFP^{TetO.KRAB} were determined by standard qPCR using the following materials and methods. Cells were harvested using trypsin after 48hr treatment with the selected compounds. The total RNA was extracted using the Maxwell LEV simply RNA Cells Kit (Promega, Madison, USA). The concentrations of RNA were detected using NanoDrop 1000 Spectrophotometer (Thermo Fisher Scientific, Waltham, USA). cDNA was obtained using 500 ng total RNA by the Reverse Transcription kit (Promega, Madison, USA) following the instructions in the manual and using primers described in Reference (3). The qPCR amplifications were performed in an ABI Prism 7900HT Sequence Detection System. The protocol was as follows: incubation at 95°C for 10 min, followed by 40 rounds with at 95°C for 15 seconds, at 60°C for 1 min. Subsequently, the samples were incubated at 95°C for 15 seconds, at 60°C for 1 min, at 95°C for 15 seconds. Data were analysed using QuantStudioTM Real-Time PCR software (ThermoFisher Scientific, USA). It was found that D3 and H6 (0.1 µM) did not significantly influence the expression level of gRNA in both H27 and HEK:EGFP^{TetO.KRAB}.

### 3.4 Effects of D3 and H6 on AdV-Cas9 expression and adenovirus cellular receptor on CD46 protein expression

Effects of D3 and H6 on AdV-Cas9 expression and adenovirus cellular receptor on CD46 protein expression were determined by western blotting and flow cytometric analysis as described in Nucleic Acids Res., 48(2): 517-532 (doi: 10.1093/nar/gkz1136). It was found that D3 and H6 (10 µM) did not significantly influence CAS9 expression level in H27, and CD46 expression level in both H27 and HEK.EGFP^{TetO.KRAB}.

### 3.5 Effects of D3 and H6 on plasmid Cas9 expression

Effects of D3 and H6 on plasmid Cas9 expression in H27 and HEK.EGFP^{TetO.KRAB} were determined as follows. Cells were collected using trypsin and lysed using RIPA buffer contains PIC (Protease Inhibitor Cocktail; Thermo Fisher Scientific, USA). The concentrations of protein were determined using a Pierce BCA Protein Assay Kit (Thermo Fisher Scientific, USA). Samples were separated by running pre-cast SDS-PAGE Gels (Bio-Rad, Hercules, USA) and then transferred by a PVDF membrane (Polyvinylidene Difluoride) on ice. The PVDF membrane was blocked with PBST contains 5% skimmed milk (0.1% Tween-20) at room temperature for 1 h. The blocked membrane were incubated with primary antibodies at 4°C overnight. After washing three times with the PBST, the membrane was incubated with secondary antibodies conjugated with HRP to detect protein by chemiluminescence using ECL (Perkin Elmer, Western Lightning Plus ECL). Images were observed using GeneSnap image analysis software (SynGene, Frederick, USA). The antibodies we used were purchased from Cell Signalling are as follows: Cas9 (#14697) and β-actin (#4970). The dilution ratio of primary antibodies was 1:1000 (v/v) and 1:10000 (v/v), respectively. The secondary antibodies including rabbit antimouse (#P0260) and goat anti-rabbit (#P0448) HRP were purchased from Dako Denmark and the secondary antibodies was diluted in a ratio of 1:2000 (v/v). The dilution of fluorescent secondary antibody (#92632211 and #92668070) was 1:10000 (v/v). All the antibodies were diluted in the PBST contains 5% BSA (Catalog#9048-46-8, Life Science). It was found that D3 and H6 (10 µM;) did not significantly influence plasmid CAS9 expression in H27 and HEK.EGFP^{TetO.KRAB}.

### 3.6 Effects of H6 on cell cycle

Effects of H6 on cell cycle of H27 and HEK.EGFP^{TetO.KRAB} were determined by propidium iodide staining using the following materials and methods. PI (Propidium Iodide, Sigma P4864-10ML). Briefly, cells were seeded at 20,000 cells/well of 6-well plates. After 24 hr incubation, the cells were treated with D3 and H6 at concentrations of from 0.01 to 10 µM. Untreated H27 and HEK.EGFP^{TetO.KRAB} (i.e. without D3 or H6) were used as control. Subsequently, cells were harvested using trypsin and washed with 1 × PBS (Phosphate Buffered Saline) twice. After centrifuging, cells were resuspended with working buffer contains PI (1mg/ml), RNase (10mg/ml) and 0.1% Triton X-100. The different phases of cell cycle were determined by flow cytometry. It was found that D3 and H6 did not significantly influence the cell cycle (G2/M, S and G0/G1 phase) of H27 and HEK.EGFP^{TetO.KRAB}.

### 3.7 Effects of D3 and H6 on Cas9 and targeted DNA binding by Chromatin immunoprecipitation (ChIP)-qPCR

Effects of D3 and H6 on Cas9 and targeted DNA binding were determined by Chromatin immunoprecipitation (ChIP)-qPCR using the following materials and methods. Chromatin immunoprecipitation (ChIP) and qPCR were performed to detect the binding of Cas9 to targeted DNA in the presence or absence of compounds. The cells were cultured in the presence or absence of compounds at 0.1 µM for 48 hr, after which cells were transfected with gRNA:Cas9 complex to target desired sequence. At three days post-transfection, cells were fixed according to the protocol as described previously(4). In brief, 1/10 of the cell culture medium volume of fresh 11% formaldehyde solution was added to the cell culture medium. The culture flasks were swirled for 15 min at room temperature. Subsequently, 1/20 of medium volume of 2.5 M glycine was used to quench the fixation process. After a 5 min swirling at room temperature, cells were harvested using a silicone scraper and transferred to a 50ml tube. The harvested cells were washed with PBS using centrifuging at 1350 ×g for 5 min at 4 °C for two cycles. Next, the nuclei were isolated using pre-chilled solution (0.1% NP-40 in PBS) and collected by centrifuging at 10,000 rpm at 4°C for 10 seconds. Furthermore, to obtain DNA fragments with suitable size from 100 to 1000 bp, MNase (Micrococcal Nuclease) was used to digest genomic DNA in terms of the manufacturer's instruction. 10% of cross-linked chromosome were incubated with Protein-A Dynabeads which were pre-coated with Cas9 antibody (mAb) (8C1-F10) at 4°C overnight. Next, the immune complexes were washed with RIPA buffer (50 mM Hepes-KOH with pH7.5, 500 mM LiCl, 1 mM EDTA with pH 8.0, 1% IGEPAL CA-630 and 0.7% Sodium Deoxycholate) and TE buffer (10 mM Tris-HCl with pH8.0 and 1 mM EDTA with pH8.0), respectively, and then eluted with Elution buffer (1% SDS and 100 mM NaHCO3) overnight at 65°C. qPCR amplifications with EGFP ORF primers targeting different regions (i.e. 5' and 3' EGFP gene segments) were performed using purified eluted DNA fragments. All of the primer sequences have been described in detail in Reference (4).. It was found that D3 and H6 significantly reduced the binding of Cas9 to targeted DNA.

### 3.8 Effects of D3 and H6 on status of chromatin by MNase-qPCR

MNAse-qPCR protocol was described elsewhere (Methods Mol. Biol. 2012; 833:63-87; and Nucleic Acids Res., 48(2): 517-532). Briefly, nuclei were isolated using ice-cold PBS containing 0.1% NP-40 supplemented with 1× PIC (cOmplete™, Mini, EDTA-free, Roche). The cell suspension was vortexed for 5s at maximum speed followed by centrifugation for 5-10 s at 9000 × g. The supernatant was discarded and the pellet was resuspended in 1 ml lysis buffer described above again to wash the nuclei. The nuclei were centrifuged for another 5-10 s at 10 000 rpm and the supernatant was again discarded. Next, the nuclei were resuspended in 100 µl MNAse buffer (50 mM Tris-HCl, 5 mM CaCl₂, 100 µg/ml BSA, pH 7.9) and warmed to 37°C, followed by addition of 5 U (unit) MNAse (NEB, diluted to 1 U/µl in MNAse buffer). 5 min digestion using MNase was stopped by adding EDTA to a final concentration of 5 mM and cooling on ice. The suspension was centrifuged for 5-10 s at 10 000 rpm and the supernatant was transferred to a new tube. Proteinase K was added to a final concentration of 1 mg/ml and incubated for 1 h at 50°C. DNA was size selected and purified with Ampure beads in terms of the manufacture's protocol (AMPure XP, A63881, Beckman Coulter, USA). The DNA was eluted in 80 µl water.

The qPCR amplifications were performed in an ABI Prism 7900HT Sequence Detection System. The protocol was described as previously. Data were analyzed using QuantStudio™ Real-Time PCR software (ThermoFisher Scientific, USA).

The data showed that D3 and H6 compounds induced an open state of chromatin in the target loci.

### 3.9 Effects of D3 and H6 on purified CRISPR/Cas9 and gRNA mediated in vitro DNA cleavage

The effect of different concentrations of D3 and H6, namely concentrations of from 1 nM to 100 µM, on purified CRISPR/Cas9 and gRNA mediated *in vitro* DNA cleavage was determined as follows.

### (a) Step1. sgRNA preparation

To generate the DNA templates for sgRNA transcription, two complementary oligonucleotides, containing T7 promoter, 20 base target sequence, and 80 base scaffold sequence, were annealed. The sequences of the respective oligonucleotides for sgRNA transcription were:

| Name | Strand | Sequence |
|---|---|---|
| T7-sgRNA | F | 5'- TAATACGACTCACTATAGGGAGCGCACCATCTTCTTCAGTTTT AGAGCTAGAAATAGCAAGTTAAAATAAGGCTAGTCCGTTATC AACTTGAAAAAGTGGCACCGAGTCGGTGCTTTTTT-3' |
| | R | 5'- AAAAAAGCACCGACTCGGTGCCACTTTTTCAAGTTGATAACG GACTAGCCTTATTTTAACTTGCTATTTCTAGCTCTAAAACTGA AGAAGATGGTGCGCTCCCTATAGTGAGTCGTATTA-3' |

sgRNAs were transcribed by a MEGAscript™ T7 Transcription Kit (Thermo Fisher Scientific , Waltham, USA), followed by purification with a MEGAclear™ Transcription Clean-Up Kit (Thermo Fisher Scientific , Waltham, USA) according to manufacturer's protocol. The concentration of sgRNA was determined using NanoDrop 1000 Spectrophotometer (Thermo Fisher Scientific, Waltham, USA).

### (b) Step2. Cas9 and dCas9 protein expression and purification

To express hCas9 protein, the pET-28b-Cas9-His plasmid (Addgene plasmid #47327)was transformed into competent BL21(DE3) strain growing in an auto-induction medium supplemented with 100µg/ml ampicillin and at 37°C for 1 hour with shaking at 250 rpm, followed by overnight incubation at 20°C as described in Reference (5). Later, cells were harvested by centrifugation and lysed with a lysis buffer (20 mM Tris-Cl, pH 8.0, 300 mM NaCl, 20 mM imidazole) by sonication at 40% duty, 20-sec pulse, 60-sec pause, for a total of 10 mins pulse, on ice water. After centrifuging at 3.8 × 10⁴ g for 60 mins at 4°C, the soluble protein was purified from the supernatant by a column containing Ni-NTA agarose resin (Qiagen, Hilden, Germany). After a 30-column-volume harsh wash with lysis buffer, the column-bound protein was eluted with an elution buffer (20 mM Tris-Cl, pH 8.0, 300mM NaCl, 500 mM imidazole). Sequentially, the Ni-NTA column purified hCas9 protein were future purified by an AKTA go protein purification system using a the Superdex 200 16/60 (GE Healthcare, Pittsburgh, USA) column with an hCas9 protein elution buffer (20mM Tris-Cl, pH 8.0, 200 mM KCl, 10mM MgCl₂, 20% (v/v) glycerol) The purity of the proteins were analyzed by NuPage, and the concentration was determined by the BCA (bicinchoninic acid) Protein Assay Kit (Pierce, Rockford IL, USA), according to the manufacturer's instructions. The single-use aliquots were kept at -80 °C.

### (c) Step3. In vitro DNA cleavage assay

The *in vitro* DNA cleavage assay was performed to detect the inhibition effect of compounds on DNA cleavage using CRISPR/Cas9. Purified Cas9 protein was incubated with selected compounds for 20 min at room temperature. Subsequently, sgRNA and EGFP template were added and incubated for 1 h at 37°C. After incubation, Cas9 protein was degraded using Proteinase K (REF#03115828001, Sigma) for additional 30 min at 56°C. The mixtures were then measured by running a DNA agarose gel. Images were captured using GeneSnap image analysis software (SynGene, Frederick, USA) and quantified and analyzed by ImageJ Software (National Institute of Health, USA).

It was found that H6 inhibited the Cas9 DNA cleavage activity in a dose dependent manner at non-toxic working concentrations.

### Example 4 - General synthesis of 1,1'-(5-amino-3-(hetero)aroyl-1H-pyrrole-2,4-diyl)bis((hetero)aroyl-1-one)

The synthesis follows the method previously described by us, Hao Guo et al., European Journal of Medicinal Chemistry, Volume 139, 2017, 633-643. Photoactivation provides a mechanistic explanation for pan-assay interference behaviour of 2-aminopyrroles in lipoxygenase inhibition.

The synthesis of 2-aminopyrrole analogues was based on a multicomponent reaction by refluxing a solution of three reactants including a methylsulfonamidoacetophenone, aldehydes and a cyanoacetic acid with 0.6 equiv of K₂CO₃ in ethanol.

### Example 5 - Synthesis of (5-amino-4-(1H-indole-3-carbonyl)-3-(3-(trifluoromethyl)phenyl)-1H-pyrrol-2-yl)(phenyl)methanone and (5-Amino-4-(5-fluoro-1H-indole-3-carbonyl)-3-(pyridin-2-yl)-1H-pyrrol-2-yl)(phenyl)methanone

### 5.1 Synthesis of 4-methyl-N-(2-oxo-2-phenylethyl)benzenesulfonamide

To a solution of aminoacetophenone hydrochloride (5.16g, 30 mmol) and DMAP (0.36 g, 3 mmol) in DCM (90 mL)were added triethylamine (7.59 g, 75 mmol) and TsCl (5.73g, 30 mmol) at 0 °C under argon. The reaction mixture was allowed to warm to room temperature naturally without removing the ice-bath over a period of 6h. Then, saturated NH₄Cl solution was added and the mixture was extracted with dichloromethane. The organic phase was separated, washed with brine and dried over Na₂SO₄. The solvent was evaporated under the reduced pressure and the residue was purified by column chromatography on silica gel (eluent: petroleum ether: ethyl acetate: dichloromethane = 3:1:1) and then recrystallized from EtOH (20 mL) to give 2.74g of a yellow solid.

Yield: 30.6%. ¹H NMR (500 MHz, CDCl₃) *δ* 7.89 - 7.85 (m, 2H), 7.84 - 7.78 (m, 2H), 7.65 - 7.59 (m, 1H), 7.49 (t, *J* = 7.9 Hz, 2H), 7.31 (d, *J* = 8.4 Hz, 2H), 5.72 (t, *J* = 4.6 Hz, 1H), 4.48 (d, *J* = 4.6 Hz, 2H), 2.41 (s, 3H). ¹³C NMR (126 MHz, CDCl₃) *δ* 192.56, 143.79, 136.09, 134.45, 133.78, 129.85, 128.99, 127.89, 127.20, 48.69, 21.53.

### 5.2 Synthesis of 3-cyanoacetylindole

Indole (1.17 g, 10 mmol) was added to a solution prepared by dissolution of cyanoacetic acid (1.02 g, 12 mmol) in Ac₂O (10 mL) at 50 °C. The solution was heated at 85 °C for 5 minutes. During that period 3-cyanoacetylindole started to crystallize. After 5 more minutes, the mixture was allowed to cool and the solid was collected, washed with MeOH and dried to give 1.4g of a yellow solid.

Yield : 76.1%.¹H NMR (500 MHz, DMSO-*d₆*) *δ* 12.19 (s, 1H), 8.38 (d, *J* = 3.1 Hz, 1H), 8.14 (dd, *J* = 6.8, 2.0 Hz, 1H), 7.54 - 7.48 (m, 1H), 7.29 - 7.20 (m, 2H), 4.50 (s, 2H). ¹³C NMR (126 MHz, DMSO-*d₆*) *δ* 183.36, 137.11, 135.95, 125.64, 123.79, 122.80, 121.49, 116.90, 114.92, 112.90, 29.89.

### 5.3 Synthesis of (5-amino-4-(1H-indole-3-carbonyl)-3-(3-(trifluoromethyl)phenyl)-1H-pyrrol-2-yl)(phenyl)methanone

3-(Trifluoro)benzaldehyde, (348 mg, 2 mmol), 4-methyl-N-(2-oxo-2-phenylethyl)-benzenesulfonamide (596.7 mg, 2mmol), 3-cyanoacetylindole (368.4 mg, 2 mmol), triethylamine (202 mg, 2 mmol) were placed in 10mL 2,2,2-trifluoroethanol in a 20-mL vial with a stir bar. The reaction was allowed to heat to 70 °C in an oil bath for 12h. Then, the reaction was cooled down to 0°C to yield a precipitate that is filtered. The solid is washed with cold ethanol and dried in an oven to yield the desired products as light-yellow solid. The solid recrystallized from ethanol to give 484 mg yellow crystals.

Yield: 51.1%.¹H NMR (500 MHz, DMSO) δ 11.26 (s, 1H), 11.08 (s, 1H), 7.98 - 7.91 (m, 1H), 7.24 - 7.18 (m, 1H), 7.18 - 7.11 (m, 3H), 7.11 - 7.01 (m, 6H), 6.94 (dt, J = 14.9, 7.7 Hz, 3H), 6.19 (s, 2H). ¹³C NMR (126 MHz, DMSO) 186.13, 184.55, 149.17, 139.46, 136.27, 136.01, 134.73, 133.77, 132.03, 130.35, 128.70, 128.01, 127.88, 127.61, 126.25, 125.30, 123.12, 122.53, 121.42, 121.38, 121.30, 121.19, 116.66, 112.04, 108.82.

### 5.4 Synthesis of 3-(5-Fluoro-1H-indol-3-yl)-3-oxopropanenitrile

The title compound was prepared from 5-fluoro-indole (.35g, 10 mmol) in accordance with the procedure described for 3-cyanoacetylindole in section 5.2 above. 445 mg of the compound were obtained as grey solid.

Yield:22.1%.¹H NMR (500 MHz, DMSO-*d₆*) δ 12.30 (d, *J* = 0.9 Hz, 1H), 8.43 (d, *J* = 3.2 Hz, 1H), 7.80 (q, *J* = 2.7 Hz, 1H), 7.53 (dd, *J* = 8.8, 4.5 Hz, 1H), 7.15 - 7.03 (m, 1H), 4.51 (s, 2H). ¹³C NMR (126 MHz, DMSO-*d₆*) *δ* 183.45, 159.31 (d, *J* = 235.4 Hz), 137.35, 133.68, 126.24 (d, *J* = 11.2 Hz), 116.77, 114.96 (d, *J* = 4.5 Hz), 114.30 (d, *J* = 9.9 Hz), 111.99 (d, *J* =25.8 Hz), 106.36 (d, *J* = 22.3 Hz), 29.90.

### 5.5 Synthesis of (5-Amino-4-(5-fluoro-1H-indole-3-carbonyl)-3-(pyridin-2-yl)-1H-pyrrol-2-yl)(phenyl)methanone

2-Pyridinecarboxaldehyde (294 mg, 2 mmol), 4-methyl-*N*-(2-oxo-2-phenylethyl)benzenesulfonamide (596.7 mg, 2mmol), 3-(5-fluoro-1*H*-indol-3-yl)-3-oxopropanenitrile (404.4 mg, 2 mmol), triethylamine (202 mg, 2 mmol) and 0.2 M 2,2,2-trifluoroethanol (10 mL) were stirred in a 20-mL vial. The reaction was allowed to heat to 70 °C in an oil bath for 12h. After the reaction, the mixture was purified by silica chromatography (MeOH/DCM=3:100) to give the target compound. 55 mg. Yellow solid.

Yield:6.5%. ¹H NMR (500 MHz, Methanol-*d4*) δ 7.86 - 7.77 (m, 1H), 7.65 (dd, *J* = 9.8, 2.5 Hz, 1H), 7.22 (d, *J* = 7.6 Hz, 2H), 7.18 - 7.09 (m, 3H), 7.04 - 6.91 (m, 4H), 6.87 (td, *J* = 9.1, 2.5 Hz, 1H), 6.78 (dd, *J* = 7.4, 5.1 Hz, 1H).

### References

1. Gonçalves,M.A.F. V, van der Velde,I., Knaän-Shanzer,S., Valerio,D. and de Vries,A.A.F. (2004) Stable transduction of large DNA by high-capacity adeno-associated virus/adenovirus hybrid vectors. Virology, 321, 287-96.
2. Janssen,J.M., Chen,X., Liu,J. and Gonçalves,M.A.F.V. (2019) The Chromatin Structure of CRISPR-Cas9 Target DNA Controls the Balance between Mutagenic and Homology-Directed Gene-Editing Events. Mol. Ther. - Nucleic Acids, 16, 141-154.
3. Bornelöv,S., Reynolds,N., Xenophontos,M., Gharbi,S., Johnstone,E., Floyd,R., Ralser,M., Signolet,J., Loos,R., Dietmann,S., et al. (2018) The Nucleosome Remodeling and Deacetylation Complex Modulates Chromatin Structure at Sites of Active Transcription to Fine-Tune Gene Expression. Mol. Cell, 71, 56-72.e4.
4. Chen,X., Rinsma,M., Janssen,J.M., Liu,J., Maggio,I. and Gonçalves,M.A.F. V (2016) Probing the impact of chromatin conformation on genome editing tools. Nucleic Acids Res., 44, 6482-92.
5. Abdallah,I.I., van Merkerk,R., Klumpenaar,E. and Quax,W.J. (2018) Catalysis of amorpha-4, 11-diene synthase unraveled and improved by mutability landscape guided engineering. Sci. Rep., 8, 9961.
6. Jinek,M., Chylinski,K., Fonfara,I., Hauer,M., Doudna,J.A. and Charpentier,E. (2012) A programmable dual-RNA-guided DNA endonuclease in adaptive bacterial immunity. Science (80-.)., 337, 816-821.

## Claims

1. Use of a compound of formula (I): 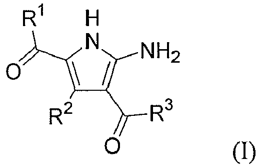 wherein
R¹ is an aryl or heteroaryl group, which groups may optionally be substituted;
R² is an aryl or heteroaryl group, which groups may optionally be substituted; and
R³ is a group of formula 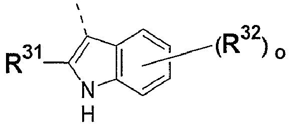
wherein
o is 1 or 2;
R³¹ is a hydrogen atom, deuterium atom, halogen atom, OH, an optionally substituted C₁-C₆ alkyl or (C₁-C₆)heteroalkyl;
R³² is independently selected at each occurrence from hydrogen atom, deuterium atom, halogen atom, or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
or a salt thereof, as an inhibitor for the activity of a RNA guided endonuclease in a basic research, biomedical or biotechnological application.

2. The use according to claim 1, wherein R¹ is an optionally substituted aryl group.

3. The use according to claim 1 or 2, wherein R² is an optionally substituted phenyl group, or an optionally substituted heteroaryl group containing 5, 6, 9 or 10 ring atoms.

4. The use according to any one of claims 1 to 3, wherein R¹ is a group of formula 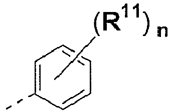 wherein
n is 1, 2, or 3, and
R¹¹ is independently selected at each occurrence from halogen atom, deuterium atom, OH, a C₁₋₆ alkyl group or a C₁₋₆ heteroalkyl group.

5. The use according to any one of claims 1 to 4, wherein R² is a group of formula 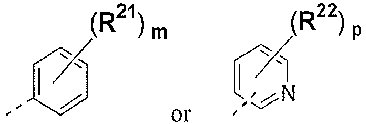 wherein
m is 1, 2, or 3;
p is 1, 2, or 3;
R²¹ is independently selected at each occurrence from halogen atom, deuterium atom, OH, a C₁₋₆ alkyl group or a C₁₋₆ heteroalkyl group; and
R²² is independently selected at each occurrence from halogen atom, deuterium atom, OH, a C₁₋₆ alkyl group or a C₁₋₆ heteroalkyl group.

6. The use according to any one of claims 1 to 5, wherein the application is performed in a cell.

7. A method of inhibiting the activity of an RNA guided endonuclease, the method comprising contacting a compound of formula (I): 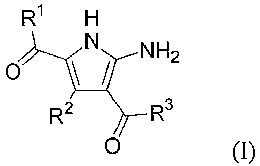 as defined in any of claims 1 to 5 with an RNA guided endonuclease.

8. The method of claim 7, wherein the compound and the RNA guided endonuclease are contacted in a cell.

9. The use of claim 6, or the method of claim 8, wherein the cell is a prokaryotic or eukaryotic cell.

10. The use or method of claim 9, wherein the prokaryotic cell is a bacterium cell.

11. The use or method of claim 9, wherein the eukaryotic cell is a human, mammalian, insect, plant, or yeast cell.

12. The use or method of any one of claims 8 to 11, wherein the cell is in an organism.

13. The use or method of any one of claims 1 to 12, wherein the RNA guided endonuclease is Cas9 or Cpf1.

14. A compound as defined in any one of claims 1 to 5, or a pharmaceutical composition comprising a compound as defined in any one of the preceding claims or a pharmaceutically acceptable salt, solvate or hydrate thereof, optionally in combination with a pharmaceutically acceptable carrier and/or adjuvant, for use in the treatment or prevention of a bacterial infection, or a genetic disease.


SEQUENCE LISTING

<110>  Rijksuniversiteit Groningen

<120>  Small molecule inhibitors of RNA guided endonucleases

<130>  23179 EP

<160>  2

<170>  PatentIn version 3.5

<210>  1
<211>  120
<212>  DNA
<213>  Artificial Sequence

<220>
<223>  Forward Primer 5'-3' for sgRNA transcription

<400>  1
taatacgact cactataggg agcgcaccat cttcttcagt tttagagcta gaaatagcaa      60

gttaaaataa ggctagtccg ttatcaactt gaaaaagtgg caccgagtcg gtgctttttt     120


<210>  2
<211>  120
<212>  DNA
<213>  Artificial Sequence

<220>
<223>  Reverse Primer 5'-3' for sgRNA transcription

<400>  2
aaaaaagcac cgactcggtg ccactttttc aagttgataa cggactagcc ttattttaac      60

ttgctatttc tagctctaaa actgaagaag atggtgcgct ccctatagtg agtcgtatta     120